# EUROPEAN PATENT APPLICATION

(11) **EP 4 454 544 A1**
(43) Date of publication of application: **30.10.2024**
(21) Application number: 23382395.4
(22) Date of filing: 27.04.2023
(51) Int. Cl.: A61B 5/00

(54) **A METHOD AND SYSTEM FOR DETECTION OF A PERISTALSIS EVENT**

(71) Applicant: Sonda Devices S.L., 46004 Valencia (ES)
(72) Inventor: MIRA TOMÁS, Juan Miguel, 46005 Valencia (ES); ALBEROLA RUBIO, José, 46017 Valencia (ES); DÍAZ MARTÍNEZ, Alba, 46530 Puzol (ES); GARCÍA CASADO, Francisco Javier, 46023 Valencia (ES)
(74) Representative: ZBM Patents - Zea, Barlocci & Markvardsen

(57) **Abstract**

There is provided a method for detecting a peristalsis event in a time-dependent signal, the method comprising the steps of:
- retrieving at least one input signal, the input signal comprising a transformation of the time-dependent signal to the frequency domain, the time-dependent signal comprising an electro hysterogram, EHG; and
- detecting a peristalsis event by processing the at least one input signal by a computer-implemented machine-learning, ML, model, which has been trained in a supervised manner using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG.

## Description

The present disclosure relates to methods and systems used for the estimation of alterations in a signal representing activity in a myometrial tissue.

### BACKGROUND

An electro hysterogram, EHG, signal is a signal related to action potentials propagating through the myometrium to the abdomen of women which is also known as uterine contraction signal. Information such as the frequency of uterine contractions, length of the contraction and contraction power of uterus, may help to derive a condition of the uterus.

Some systems for monitoring uterine contractions may comprise electrodes and computing devices to receive EHG signals from the electrodes and to transform the EHG signals into uterine potential contraction events. Some methods to detect contractions events may be improved.

There is a need for a method to accurately detect peristalsis or contraction events.

### SUMMARY

The present disclosure provides a computer-implemented method for detecting a peristalsis event in a time-dependent signal. The method comprises the steps of:
- retrieving at least one input signal, the input signal comprising a transformation of the time-dependent signal to the frequency domain, the time-dependent signal comprising an electro hysterogram, EHG; and
- detecting a peristalsis event by processing the at least one input signal by a computer-implemented machine-learning, ML, model, which has been trained in a supervised manner using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG.

The method described herein improves the state-of-the-art methods which rely on statistical data and may lead to unjustified assumptions, for example, methods of the prior art may establish a frequency threshold upon which, every frequency event is considered a peristalsis event. The methods of the present disclosure allow that a trained model identifies signal features leading to a more comprehensive analysis of the EHG input signals.

The ML model ingests a spectral representation of a time-domain signal comprising an EHG. The spectral representation or transformation to the frequency domain may be discrete, or sampled or may be formed of discrete samples. The "at least one input signal" may be discrete or sampled so that the ML processes a sampled or discrete input signal. In the present disclosure "transformations of time-dependent training signals to the frequency domain" may comprise the spectral components of a time-dependent signal, which may be acquired by, for example, acquiring a Power Spectral Density, PSD, of the time-dependent signal. The spectral components may be acquired by, for example, acquiring the Fourier transformation, FFT or DFT, of a discrete or analog time dependent signal.

The ML model provides a probability that the time-domain signal comprising the EHG comprises a peristalsis event based on the spectral representation or the spectral components of the time-domain or time-dependent signal. The method of the present disclosure advantageously provides an alternative method which may be used for the estimation of alterations or conditions in a myometrial tissue. For example, signals comprising one or more EHG signals of a non-pregnant uterus may be processed by the methods of the present disclosure to provide a probability or estimation of peristalsis events in the signal. For example, signals comprising one or more EHG signals of a pregnant uterus may be processed by the methods of the present disclosure to provide a probability or estimation of peristalsis events in the signal.

Retrieving at least one input signal may comprise receiving one or more time domain signals from a data acquisition device or from a database; and obtaining or computing the transformation of the time-dependent signals to the frequency domain. A transformation of the time-dependent signals to the frequency domain allows obtaining a frequency-domain signal.

Detecting a peristalsis event by processing the input signals by a computer-implemented ML model advantageously allows providing a method to detect a peristalsis event based on a comprehensive analysis of the input signals which results in an improved detection of peristalsis events, including peristalsis patterns.

The method may further comprise predicting whether a subject or a person is likely to present or develop a condition based on the detection of peristalsis event or events. Particularly, conditions related to fertility status or conditions may be derived from the detection of peristalsis events in the retrieved at least one input signal comprising an EHG signal.

The method may further comprise performing the transformation of the time-dependent signal to the frequency domain. The transformations of time-dependent training signals to the frequency domain may comprise performing or computing a Power Spectral Density, PSD, of the time-dependent signal. The time dependent signal may be processed by the methods of the disclosure. For example, if a time-dependent signal is stored in a database, the time-dependent signal may be sampled or may be received already sampled. The methods of the disclosure may comprise selecting a time-window of the sampled time-dependent signal and computing the PSD of the time-window. In other words, a group of time-domain samples may be selected and a PSD of the group of time-domain samples may be computed. The PSD may be computed with N number of frequency bins, e.g., 32, 64, 128, 256, 512, 1024 points.

In some examples the method comprises using an ML model which comprises an input layer comprising a predefined number of consecutive samples, and the method comprises:
- forming a group of a predefined number of consecutive samples of the transformation of the time-dependent signal to the frequency domain; and
- inputting the group of consecutive samples into the input layer of the ML model, wherein the ML model comprises an input layer comprising at least the said predefined number of neurons, and wherein each of the consecutive samples are input into a respective neuron.

Forming a group of a predefined number of consecutive samples of the frequency-domain signal may comprise selecting at least a portion of the real part of a group of real and imaginary values of a frequency-domain signal. When computing, for example, the PSD of a time-dependent signal, then, real values, and imaginary values of the PSD are obtained. Forming a group may comprise selecting at least some of the real values.

Inputting the group or formed group of consecutive samples comprises inputting a number of consecutive samples into an input layer of the ML model, the input layer comprising at least the predefined number of neurons, and wherein each of the consecutive samples are input into a respective neuron. Each of the respective neurons may comprise consecutive neurons. In other words, a number of consecutive samples of the frequency-domain signal are input in batch and in parallel into the input layer of the ML model. Each of the consecutive frequency-domain samples are input into the input layer of the ML model, the input layer comprising at least the number of neurons, and wherein each of the consecutive samples are input into a respective neuron. The inputting may be iterated for a new group of samples wherein the new group of samples may be obtained by shifting a number of samples the group of consecutive samples, for example shifting 1 or more samples the group of consecutive samples of the frequency-domain signal. Inputting the group or formed group of consecutive samples at once into the input layer of the ML model advantageously provides a near real time estimation or inference of the probability that the group of consecutive frequency-domain samples correspond to a time-window of a sampled time-dependent signal containing, or not, a peristalsis event.

The method may further comprise performing a supervised training of the ML model, using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG. The training is supervised, and a ML model may be used such as, for example, a neural network or a support vector machine. The result provided by the trained method is a probability that the time-dependent signals from which the transformation to frequency domain is computed, comprises a peristalsis event.

The training pairs may be generated by receiving time-dependent EHG training signals from a repository database and generating a transformation of the time-dependent EHG training signals to the frequency domain, for example by a Power Spectral Density. For example, the transformation may comprise a Power Spectral Density, PSD, of size N or number of frequency bins, eg: PSD of size 256, or PSD of size 512, or PSD of size 1024. The time-dependent EHG training signals may have been stored in a digital database. Analog time-dependent EHG training signals may have been sampled previously to being stored in a database. A sampling frequency of 5Hz or 6Hz, or 10Hz or 15Hz or 20Hz may have been used as sampling frequency to store digitized time-dependent EHG training signals.

The acquisition of analog time-dependent signals does not form part of the present disclosure.

The quality of the data used for training the ML plays a decisive role in the accuracy of the results provided by the ML model when evaluating EHG input signals. The quality of the data may be improved if, for example, the acquired data are acquired by a multipolar EHG device, for example a bipolar EHG acquisition device, which may provide a plurality of measures related to the electric activity of myometrium organ or organs. Techniques to acquire the time-dependent EHG training signals do not form part of the present disclosure and may include non-invasive techniques.

EHG time-dependent training signals may be obtained from data in an existing database and may be divided into a training set, a validation set and a test set. The training set may comprise a 70% of the training signals, the validation set may comprise a 15% of the training signals and the test set may comprise a 15% of the training signals.

In some examples, the training comprises:
- receiving a plurality of time-dependent training signals from a database; and
- obtaining or computing a plurality of transformations of at least the plurality of time-dependent training signals to the frequency domain; and
- associating a ground-truth result, or label, to each of the transformations of the plurality.

The sampling rate of the time-domain signals may be defined at 5Hz, 10Hz or 20Hz. A group of samples of the time-domain samples may be processed and a transformation to the frequency domain may be acquired. Then, a group of frequency-domain samples may be labelled as containing a peristalsis event or as not containing a peristalsis event. Visual inspection techniques or other detection methods may be used to determine whether the training frequency-domain group of samples contain, or not, a peristalsis event so that the training frequency-domain group of samples may be labelled as containing, or not, a peristalsis event for training. The group of frequency-domain samples may be inputted into the ML model to be trained and the corresponding label or ground truth result may be inputted to the ML model such that the ML model is trained with such result or label.

Considering that a peristalsis event is defined by a frequency of [0,1 Hz - 4Hz], some techniques of the prior art may assume a threshold above which a peristalsis is detected, for example, 1 Hz. Such techniques would, therefore, consider that a signal over 1Hz represents a peristalsis event. The methods according to the present disclosure use a trained ML model to read the frequency components of the input training signals so that peristalsis are detected without assuming a peristalsis over a predetermined threshold, or at predetermined parameters.

The methods according to the disclosure provide a more accurate and precise estimation of the presence of a peristalsis event in one or more EHG input signals compared to other signal processing methods in the prior art.

Advantageously, the methods of the present disclosure can adapt to a contractile wave signal characterized by a downward-propagated triple descending gradient behavior (TGD, 1951, Caldeyro Barcia and Álvarez). Following the behavior of downward-propagated triple descending gradient contractile wave signals, the fact of parallel-inputting the group of consecutive frequency-domain samples which are correlated by said behavior provides a specific adaptation to the evolution of the contractile wave through a uterus.

During training, some examples of the method comprise providing a plurality of sampled time-dependent training signals and the method further comprises, after obtaining or computing the transformation of at least one training signal of the plurality of time-dependent training signals to the frequency domain, shifting the at least one training signal by one sample; and obtaining or computing the transformation of the shifted at least one time-dependent training signal. A time-window of samples may not contain a peristalsis event, whereas a shifted window may contain a peristalsis event. Shifting by one sample a time-window of samples and labelling the corresponding transformation to the frequency domain as containing, or not, a peristalsis event allows the ML model to differentiate more scenarios and provides more accuracy to the detection of a peristalsis event.

In some aspects, the disclosure presents a computer program product comprising instructions which, when the program is executed by a computer, cause the computer to carry out the steps of a method according to the disclosure.

The computer program may be executed in a computer such that an input interface in the computer acquires input signals which are processed, and the computer may output a result indicating the presence or probability of the presence of a peristalsis event in the input signals. The output may be recorded in a digital file or may be shown in a graphical interface. The computer program product may be embodied on a storage medium (for example, a CD-ROM, a DVD, a USB drive, on a computer memory or on a read-only memory) or carried on a carrier signal (for example, on an electrical or optical carrier signal). The computer program may be in the form of source code, object code, a code intermediate source and object code such as in partially compiled form, or in any other form suitable for use in the implementation of the processes. The carrier may be any entity or device capable of carrying the computer program. For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means. When the computer program is embodied in a signal that may be conveyed directly by a cable or other device or means, the carrier may be constituted by such cable or other device or means. Alternatively, the carrier may be an integrated circuit in which the computer program is embedded, the integrated circuit being adapted for performing, or for use in the performance of, the relevant methods.

In some aspects, the present disclosure presents a system comprising a processor and a non-transitory computer readable media in communication with the processor that stores instruction code, which when executed by the processor, causes the processor to provide an output or result indicating the presence of one or more contractions or peristalsis events in an input signal. The processor is configured to implement the steps of any of the methods of the disclosure. In some examples, the system comprises an input interface for retrieving one or more input signals from a local or remote repository database.

In some examples, once a number of samples have been input to the ML model, the ML model may provide an output indication of the probability that the inputted temporal window defined by the inputted samples comprises a peristalsis event. In such examples, a processor may provide an indication that the predefined number of inputted consecutive samples comprises a peristalsis event.

In some examples the system comprises the ML model to be trained or which has been trained. In particular the non-transitory computer readable media comprises the ML model.

The above description is only an overview of the technical solution of the present invention. In order to understand the technical means of the present invention more clearly, it can be implemented in accordance with the content of the specification, and in order to make the above and other objectives, features and advantages of the present invention more obvious and understandable. In the following, the preferred embodiments are cited in conjunction with the drawings, and the detailed description is as follows.

### DESCRIPTION OF THE FIGURES

Figure 1 shows a graphical representation of an embodiment of a method 100 according to the present disclosure;
Figure 2 shows a graphical representation of an embodiment of a method 200 according to the present disclosure;
Figure 3 shows a graphical representation 300 of a training process of a ML model according to the present disclosure;
Figure 4 shows a system 400 for detection of a peristalsis event according to the present disclosure;
Figure 5 shows a system 500 for detection of a peristalsis event according to the present disclosure.
Figure 6 shows an example of a computer-implemented method 600 for detecting a peristalsis event according to the present disclosure.
Figure 7 shows an example representation of a time-dependent signal 701 comprising an EHG and a superposed probability signal 702 provided by a system configured to carry out the steps of the methods according to the present disclosure.

### DETAILED DESCRIPTION

In order to further illustrate the technical means and effects of the present invention to achieve the intended purpose of the invention, the specific implementation, structure, features and effects of the present invention will be described in detail below with reference to the accompanying drawings and preferred embodiments.

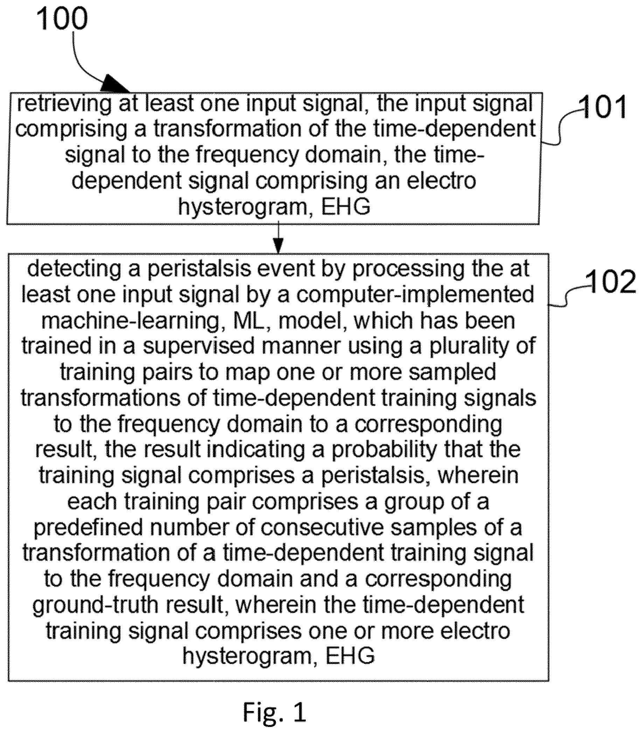

Figure 1 shows a graphical representation of an embodiment of a computer implemented method 100 according to the present disclosure. Figure 1 shows, in block 101, retrieving one or more input signals, the input signals comprising a transformation of time-dependent signals to the frequency domain, the time-dependent signals comprising an electro hysterogram, EHG. Figure 1 shows, in block 102, detecting a peristalsis event by processing the input signals by a ML model, which has been trained in a supervised manner using a plurality of training pairs to map sampled transformation of time-dependent training signals to the frequency domain to a corresponding result indicating whether the training signal comprises a peristalsis, wherein each training pair comprises a group of a predefined number of consecutive samples of the transformation of time dependent training signals to the frequency domain and a corresponding ground-truth result, wherein the time-dependent training signal comprises one or more electro hysterogram, EHG.

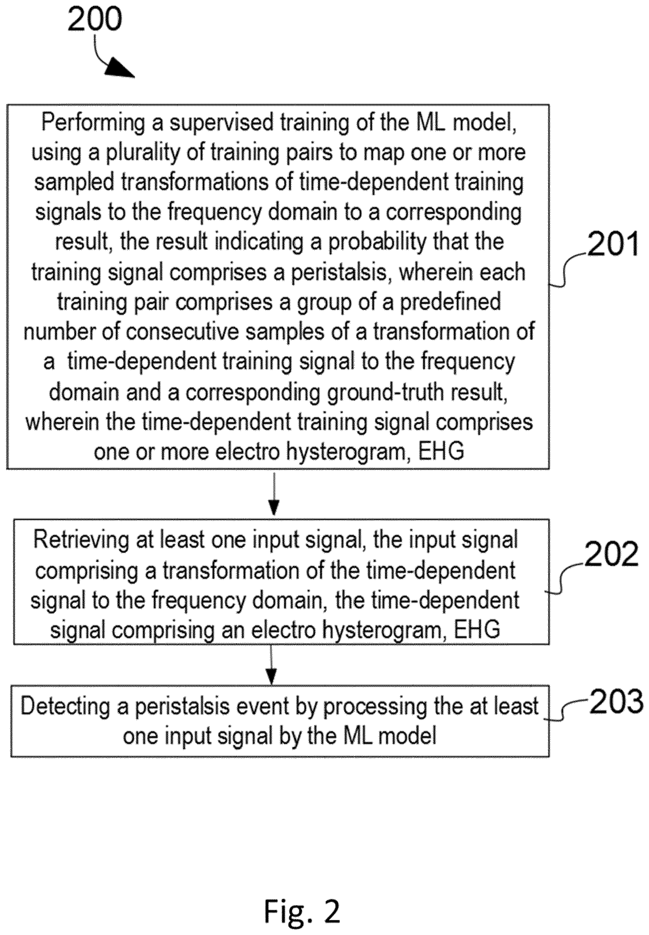

Figure 2 shows a graphical representation of an embodiment of a computer implemented method 200 according to the present disclosure. Figure 2 shows, in block 201, performing a supervised training of the ML model, using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG. Figure 2 shows, in block 202, retrieving at least one input signal, the input signal comprising a transformation of the time-dependent signal to the frequency domain, the time-dependent signal comprising an electro hysterogram, EHG. Figure 2 shows, in block 203, detecting a peristalsis event by processing the at least one input signal by the ML model.

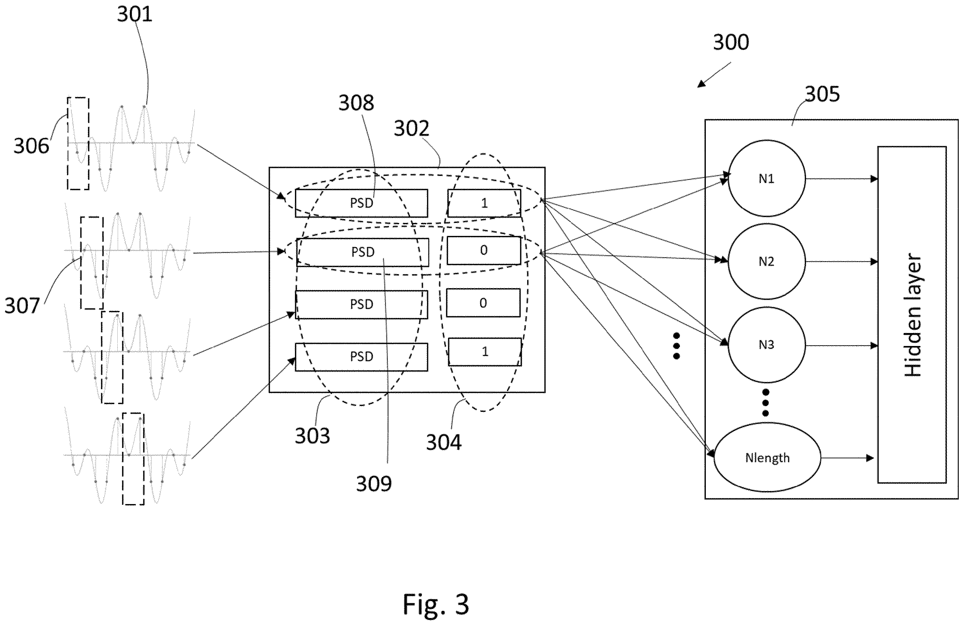

Figure 3 shows a graphical representation 300 of a training process of a ML model according to the present disclosure. A plurality of time-dependent EHG signals 301 may have been sampled and stored in a digital database. The training process of the figure 3 includes performing a supervised training of the ML model 305 by using a plurality of training pairs 302 to map one or more sampled transformations of time-dependent training signals to the frequency domain to a corresponding result, the result indicating a probability that the training signal comprises a peristalsis, wherein each training pair 302 comprises a group of a predefined number of consecutive samples of a transformation of a time-dependent training signal to the frequency domain 303 and a corresponding ground-truth result 304, wherein the time-dependent training signal 301 comprises one or more electro hysterogram, EHG, from which a transformation to the frequency domain is performed. The transformation may be a PSD of size or length, or also referred to as "frequency bins", 512. A group of 257 samples of the PSD is fed into the ML model or neural network 305, which has an input layer of 257 neurons. Each of the samples are fed consecutively into a respective neuron Ni, i=1...257.

The ground-truth results 304 or labels may have been generated based on the time-domain signal 301. A first time-window 306 of a time-domain signal which is known to comprise a peristalsis event is labelled as "1" indicating that the time-window comprises a peristalsis. Then, a PSD of the time-window 306 may be performed and the resulting signal 308 is associated to the label "1". A second time-window 307 of the time-domain signal 301 which is known to not comprise a peristalsis event is labelled as "0" indicating that the time-window does not comprise a peristalsis. Then, a second PSD of the second time-window 307 may be performed and the resulting signal 309 is associated to the label "0". The process may be repeated for a plurality of time-windows comprising and not comprising peristalsis events. Then, the training pairs are formed including each of the PSD 303 performed for each of the time-windows and the corresponding labels 304. The time windows may have a duration from 17 to 23 seconds. In some examples, the sampled time-domain signal is taken to generate a new time-window by shifting, by for example 1 sample, the time-window from which a first PSD was performed. If the time-window and the new time-window comprise a peristalsis event, then both PSD of both time window will be associated with a label indicating that there is a peristalsis event, for example by the number "1". If one of the time-windows comprises a peristalsis the PSD will be labelled as "1" and if the other does not comprise a peristalsis will be labelled as "0". The manner in which each one of the frequency samples of the PSD is inputted into the ML model may vary. A preferred embodiment comprises inputting an increasing order of frequency samples in an increasing order of neuron. For example, a frequency sample of the PSD corresponding to the lowest frequency will be inputted to the neuron N1 whereas the consecutive frequency sample, until all the 257 samples are reached, are inputted into the second neuron N2 until the neuron Nsize or neuron 257 is reached, consecutively.

The ML model of figure 3 includes a hidden layer comprising 257 neurons and an output layer with one neuron. The output layer, when the ML model 305 is trained, provides an output which comprises an indication of whether the input signal comprises a peristalsis event.

The ML model, in some examples, is iteratively trained using a supervised training using a plurality of training pairs wherein each training pair comprises a group of consecutive samples of the PSD of time-dependent training EHG signal and a corresponding ground-truth result. Preferably, during training, a maximum number of iterations is 10,000, and the learning rate or the step size at each iteration while moving toward a minimum of a loss function is 0.0001. A loss function used for training the ML model 302 of the figure 3 is sigmoid. Using this loss function, the characteristics of the EHG signal can be learned completely and quickly.

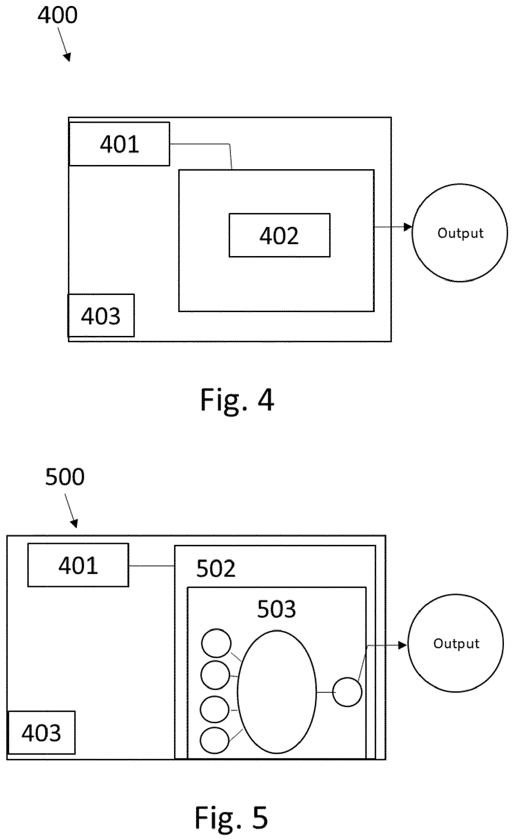

Figure 4 shows a system 400 for detection of a peristalsis event, the system comprising a processor 401 and a non-transitory computer readable media 402 in communication with the processor 401, and storing instruction code which, when executed by the processor, causes the processor to perform the steps of a method of any of the examples of the disclosure. The system 400 comprises an input interface 403 404 to receive or retrieve EHG input signals.

The system 400 may comprise communication means or interface to communicate with a remote server storing a ML trained to detect a peristalsis event in EHG signals. The readable media may comprise instructions which, when executed by the processor 401, may cause the processor 401 to communicate with the remote server and obtain a result or output indicating whether input signals comprise a peristalsis event.

Figure 5 shows a system 500 for detection of a peristalsis event, the system 500 comprising a peristalsis detection device 503 comprising a processor 401 and a non-transitory computer readable media 502 in communication with the processor 401, and storing instruction code which, when executed by the processor, causes the processor to perform the steps of a method of any of the examples of the disclosure. The system 500 differs from the system 400 in that the non-transitory computer readable media 502 stores the ML model 503, which has been trained in a supervised manner using a plurality of training pairs to map sampled transformation of time-dependent training signals to the frequency domain to a corresponding result or output indicating whether the training signal comprises a peristalsis, wherein each training pair comprises a group of a predefined number of consecutive samples of the transformation of time dependent training signals to the frequency domain and a corresponding ground-truth result, wherein the time-dependent training signal comprise one or more EHG.

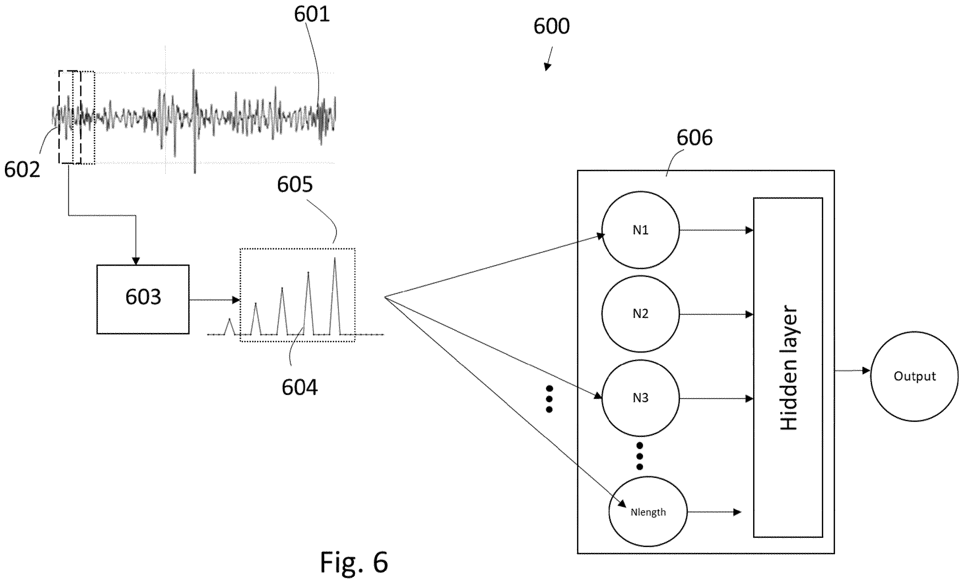

In operation, the systems of the present disclosure may operate according to the methods of the present disclosure. Figure 6 shows an example of a computer-implemented method 600 for detecting a peristalsis event in a time-dependent signal 601 implemented by the systems of the disclosure, wherein, a time-window 602 of, for example, 20 seconds, of the time-dependent signal 601 is transformed through a PSD analyzer 603. The time-dependent signal 601 comprises an electro hysterogram, EHG. Retrieving at least one input signal 604 is performed by providing at least a sampled frequency-domain signal 604 by the PSD analyzer 603. Detecting a peristalsis event is performed by processing the at least one input signal 604 by the computer-implemented machine-learning, ML, model 606, which has been trained in a supervised manner using a plurality of training pairs to map one or more sampled transformations to the frequency domain of one or more time-dependent signals to a corresponding result, the result indicating a probability that the one or more time-dependent signals comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG. The number of consecutive samples in the example of figure 6 may be comprised in the frequency window 205 and may comprise 257 samples. Then 257 samples of the PSD window 605 of the PSD signal 604 are fed into the input layer of the ML model 606. Then, detecting a peristalsis event by processing the input signal 604 by the ML model 606 comprises providing an output indicating the probability that the analyzed time-window 602 of the time-dependent signal 601 comprises a peristalsis event.

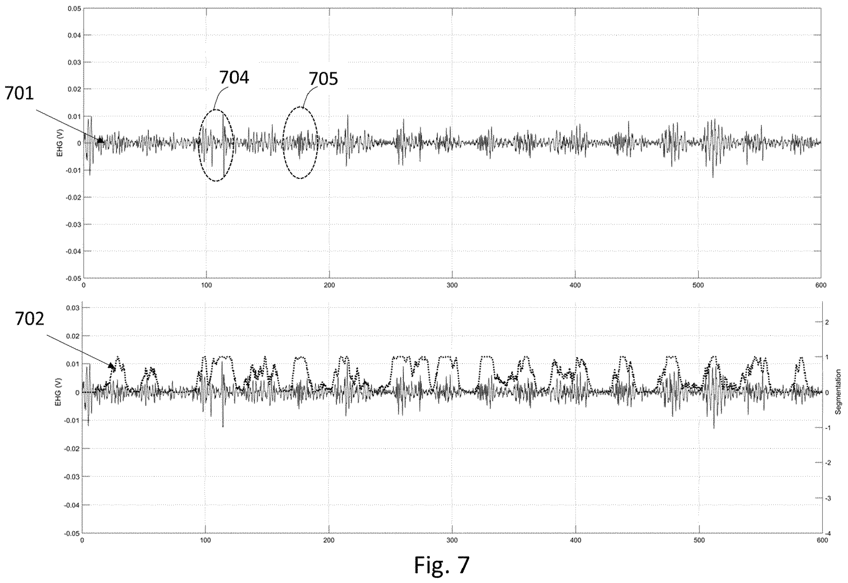

Figure 7 shows an example representation of a time-dependent signal 701 comprising an EHG and a superposed probability signal, represented as the dotted line 702, provided by a system configured to carry out the steps of the method 600, the method 600 for detecting a peristalsis event 704, 705, in a time-dependent signal 701. As seen, the methods of the present disclosure ensure accuracy in the detection of peristalsis events, sine the output probability or result 702 is 1 when a peristalsis event is detected. Methods may be provided to improve the accuracy of the detection and reduce noise. Increasing the number of training signals may provide more accurate results. As it may be understood from figure 7, the method to detect a peristalsis event may be iterated once or more times to detect a plurality of peristalsis events or patterns in one or more signals over time. The input signals may be shifted by one or more samples and the systems and methods may continuously accept or ingest new samples to be processed and to detect whether a time-dependent input signal comprises one or more peristalsis events. A particular event or a particular pattern may be used for predicting whether a subject or a person is likely to present or develop a condition based on the detection of peristalsis event or events. Particularly, conditions related to a fertility status or condition may be derived from the detection of peristalsis events or peristalsis patterns in the retrieved at least one input signal comprising an EHG signal.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples but should be determined only by a fair reading of the claims that follow. If reference signs related to drawings are placed in parentheses in a claim, they are solely for attempting to increase the intelligibility of the claim, and shall not be construed as limiting the scope of the claim.

Further, although the examples described with reference to the drawings comprise computing apparatus/systems and processes performed in computing apparatus/systems, the invention also extends to computer programs, particularly computer programs on or in a carrier, adapted for putting the system into practice.

## Claims

1. A computer-implemented method for detecting a peristalsis event in a time-dependent signal, the method comprising the steps of:
- retrieving at least one input signal, the input signal comprising a transformation of the time-dependent signal to the frequency domain, the time-dependent signal comprising an electro hysterogram, EHG; and
- detecting a peristalsis event by processing the at least one input signal by a computer-implemented machine-learning, ML, model, which has been trained in a supervised manner using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG.

2. The computer-implemented method of claim 1 wherein the retrieving at least one input signal comprises
- receiving at least one time-dependent signal from a database; and
- obtaining or computing the transformation of the time-dependent signal to the frequency domain.

3. The computer-implemented method of claim 1 or 2 further comprising performing a supervised training of the ML model, using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG.

4. The computer-implemented method of any of claims 1 to 3 further comprising:
- forming a group of a predefined number of consecutive samples of the transformation of the time-dependent signal to the frequency domain; and
- inputting the group of consecutive samples into an input layer of the ML model, wherein the ML model comprises the input layer comprising at least the said predefined number of neurons, and wherein each of the consecutive samples are input into a respective neuron.

5. The computer-implemented method of any of claims 1 to 4 further comprising performing the transformation of the time-dependent signal to the frequency domain is by computing a Power Spectral Density of the time-dependent signal.

6. The computer-implemented method of any of claims 3 to 5 further comprising:
- receiving a plurality of time-dependent training signals from a database; and
- obtaining or computing a plurality of transformations of at least the plurality of time-dependent training signals to the frequency domain; and
- associating a ground-truth result to each of the transformations of the plurality of time-dependent training signals.

7. The computer-implemented method of claim 6 wherein plurality of time-dependent training signals are sampled signals and the method further comprises, after obtaining or computing at least one of the plurality of transformations of at least one training signal of the plurality of time-dependent training signals to the frequency domain:
- shifting the at least one training signal of the plurality of time-dependent training signals by at least one sample; and
- obtaining or computing the transformation to the frequency domain of the shifted at least one training signal.

8. Computer program product comprising instructions which, when the computer program product is executed by a computer, cause the computer to carry out the steps of a computer-implemented method according to claims 1 to 7.

9. A system (400) for detection of a peristalsis event, the system comprising
- a processor (401); and
- a non-transitory computer readable media (402) in communication with the processor, and storing instruction code which, when executed by the processor, causes the processor to perform the steps of a computer-implemented method according to any of claims 1 to 7.

10. The system (500) of claim 9 wherein the non-transitory computer readable media (502) stores the ML model (503), which has been trained in a supervised manner using a plurality of training pairs to map one or more sampled transformations of time-dependent training signals to the frequency domain to a corresponding result, the result indicating a probability that the training signal comprises a peristalsis, wherein each training pair comprises a group of a predefined number of consecutive samples of a transformation of a time-dependent training signal to the frequency domain and a corresponding ground-truth result, wherein the time-dependent training signal comprises one or more electro hysterogram, EHG.
